# EUROPEAN PATENT APPLICATION

(11) **EP 4 678 205 A1**
(43) Date of publication of application: **14.01.2026**
(21) Application number: 24306146.2
(22) Date of filing: 08.07.2024
(51) Int. Cl.: A61M 5/20, A61M 5/32

(54) **AUTOINJECTOR**

(71) Applicant: Becton Dickinson France, 38800 Le Pont-de-Claix (FR)
(72) Inventor: CARREL, Franck, 38220 Saint Jean de Vaulx (FR); ALVAIN, Olivier, 38180 SEYSSINS (FR)
(74) Representative: Germain Maureau

(57) **Abstract**

This autoinjector (1) comprises an outer body (2) extending along a longitudinal axis A and configured for receiving a medical container (100) having a barrel (101) defining a reservoir for containing a medical product, said barrel (101) having a distal end (102) provided with an injection needle (103) sealed by a needle shield (104), and an opened proximal end provided with a radial flange (106). A needle cover (8) is axially movable along the longitudinal axis A between an initial position, a retracted position, proximally located with respect to the initial position, and a safety position, distally located with respect to the retracted position, in which the needle cover (8) shields the injection needle (103). A holding ring (6) is arranged within the outer body (2) for supporting the medical container (100), the holding ring (6) being axially movable between an initial position and a pricking position. The holding ring (6) is axially guided by the needle cover (8).

## Description

The present invention relates to an autoinjector.

In this application, the distal end of a component or of a device is to be understood as meaning the end furthest from the user's hand and the proximal end is to be understood as meaning the end closest to the user's hand. Likewise, in this application, the "distal direction" is to be understood as meaning the direction away from the user's hand, and the "proximal direction" is to be understood as meaning the direction toward the user's hand.

Automatic injection devices, or autoinjectors, are designed for automatic injection of a medical product into an injection site. Autoinjectors usually comprise a housing for receiving a medical container such as a syringe including an injection needle. After injection, autoinjectors are typically disposed together with the used medical container in a waste container. However, the disposal of single-use autoinjectors may have an impact on the environment.

There is therefore a need for more sustainable autoinjectors.

In this context, an aspect of the invention is an autoinjector for automatic injection of a product into an injection site, said autoinjector comprising :
an outer body extending along a longitudinal axis A and configured for receiving a medical container having a barrel defining a reservoir for containing a medical product, said barrel having a distal end provided with an injection needle sealed by a needle shield, and an opened proximal end provided with a radial flange,
a needle cover axially movable along the longitudinal axis A between an initial position, a retracted position, proximally located with respect to the initial position, and a safety position, distally located with respect to the retracted position, in which the needle cover shields the injection needle,
a holding ring arranged within the outer body for supporting the medical container, the holding ring being axially movable between an initial position and a pricking position,
wherein the holding ring is axially guided by the needle cover.

The autoinjector of the invention has a compact size in comparison with autoinjectors of the prior art. As a result, less raw material is required for manufacturing an autoinjector of the invention. This results in an improved sustainability.

The device of the invention may further include some or all of the features listed below.

In an embodiment, the holding ring includes one or more guiding members configured for complementarily engaging one or more guiding members of the needle cover such that these guiding members form a cylinder.

In an embodiment, the needle cover is axially guided by the outer body.

In an embodiment, the autoinjector includes a plunger rod axially movable between an initial position and an injection end position, distally located relative to the initial position, the plunger rod being configured to push a stopper inside the barrel in order to expel the medical product, an inner body including a retainer for retaining the plunger rod in the initial position, and an actuation button, axially movable with respect to the inner body between an initial position and an actuation position in which the actuation button engages the retainer of the inner body to release the plunger rod such that the plunger rod can move to the injection end position.

In an embodiment, the inner body is arranged within the outer body.

In an embodiment, the actuation button is arranged at a proximal end of the outer body.

In an embodiment, the autoinjector includes a provisional proximal blocking surface configured for axially abutting against the actuation button to provisionally block the actuation button in the initial position.

In an embodiment, the actuation button includes push openings.

The push openings are configured for allowing insertion of assembly fingers into the autoinjector to push the inner body in the distal direction.

In an embodiment, the inner body includes a distal stop for pushing the medical container against the holding ring.

This prevents the medical container from generating a rattling noise during handling or transport of the autoinjector.

In an embodiment, the autoinjector includes a cam, rotatably mounted within the outer body between a first angular position, a second angular position, and a third angular position, the cam including a first track engaged by the sequencing feature of the needle cover and a second track engaged by the sequencing feature of the holding ring.

In an embodiment, the cam includes an axial blocking member configured for preventing axial movement of the actuation button to the actuation position as long as the cam is in the first angular position.

That is, as long as the needle cover is not retracted in the outer body, the actuation button cannot be pressed by the user.

In an embodiment, the outer body is made of a single piece.

That is, the autoinjector has a housing formed by the outer body, and merely comprises this outer body. There is lower or bottom body. The outer body concentrates all the functions required from an autoinjector housing. The outer body supports the cap, guides the needle cover, supports the actuation button.

In an embodiment, the needle cover mainly extends beyond the distal end of the outer body.

That is, the portion of the needle cover that extends outside the outer body is axially equal to or greater than the portion of the needle cover that extends inside the outer body.

In an embodiment, the autoinjector includes a cap which is removably attached to a distal end of the outer body, and the cap extends over half the axial length of the needle cover.

This increases the distance between the distal end of the needle cover and the distal end of the outer body, thereby avoiding pinching of the patient's skin.

In an embodiment, the autoinjector includes a cap removably attached to a distal end of the outer body, and a remover arranged within the cap for removing the needle shield when the cap is withdrawn from the outer body, the cap and the remover including sequencing features configured for allowing removal of the cap first and then removal of the needle shield when the cap is detached from the outer body.

The autoinjector may include the medical container.

The invention and the advantages arising therefrom will clearly emerge from the detailed description that is given below with reference to the appended drawings as follows :
- Figure 1A is a perspective view of an autoinjector according to an embodiment of the invention,
- Figure 1B is an exploded view of an autoinjector according to an embodiment of the invention,
- Figures 2A-2B are cross-section views of an autoinjector according to an embodiment of the invention,
- Figures 3A-3B are perspective views of an outer body of an autoinjector according to an embodiment of the invention,
- Figures 4A-4C are respectively, a perspective and 90° cross-section views of an inner body of an autoinjector according to an embodiment of the invention,
- Figures 5A-5C are, respectively, a perspective and 90° cross-section views of an actuation button of an autoinjector according to an embodiment of the invention,
- Figure 6 is a perspective view of a cam of an autoinjector according to an embodiment of the invention,
- Figures 7A-7B are respectively, a perspective and a cross-section view of a holding ring of an autoinjector according to an embodiment of the invention,
- Figures 8A-8B are, respectively, a perspective and a cross-section view of a needle cover of an autoinjector according to an embodiment of the invention,
- Figure 9 is a perspective view of a cap of an autoinjector according to an embodiment of the invention,
- Figure 10 is a perspective view of a remover of an autoinjector according to an embodiment of the invention,
- Figures 11A-15 illustrate details of an autoinjector according to an embodiment of the invention,
- Figures 16-19B illustrate assembly steps of an autoinjector according to an embodiment of the invention,
- Figures 20A-25C illustrate use of an autoinjector according to an embodiment of the invention,
- Figure 26 is a perspective view of an autoinjector according to another embodiment of the invention,
- Figures 27A-27B are perspective views of a locker of an autoinjector according to this other embodiment of the invention,
- Figures 28A-28B illustrate assembly of an actuation button and a locker of an autoinjector according to this other embodiment of the invention,
- Figure 29 is a perspective view of a remover of an autoinjector according to this other embodiment of the invention,
- Figures 30A-30B are 90° cross-section views of an autoinjector according to this other embodiment of the invention,
- Figures 31A-34B illustrate details of an autoinjector according to this other embodiment of the invention,
- Figure 35 is a perspective view of a cam of an autoinjector according to an embodiment of the invention.

The different features of the embodiments can be used in combination with and used with other embodiments as long as the combined parts are not inconsistent with or interfere with the operation of the device and assembly. This invention is not limited in its application to the details of construction and the arrangement of components set forth in the following description or illustrated in the drawings. The embodiments herein are capable of being modified, practiced or carried out in various ways. The phraseology and terminology used herein is for the purpose of description and should not be regarded as limiting. The use of "including," "comprising," or "having" and variations thereof herein is to encompass the items listed thereafter and equivalents thereof as well as additional items. Unless limited otherwise, the terms "connected," "coupled," and "mounted," and variations thereof herein are used broadly and encompass direct and indirect connections, couplings, and mountings. In addition, the terms "connected" and "coupled" and variations thereof are not limited to physical or mechanical connections or couplings. In contrast, when an element is referred to as being "directly connected" or "directly coupled" to another element, there are no intervening elements present. Unless otherwise defined, all terms (including technical and scientific terms) used herein have the same meaning as commonly understood by one of ordinary skill in the art to which this disclosure belongs. It will be further understood that terms used herein should be interpreted as having a meaning consistent with their meaning in the context of this specification and the relevant art and will not be interpreted in an idealized or overly formal sense unless expressly so defined herein. Further, terms such as distal, proximal, up, down, bottom, and top are relative, and are to aid illustration, but are not limiting. Relative terms such as "below" or "above" or "upper" or "lower" or "horizontal" or "vertical" may be used herein to describe a relationship of one element to another element as illustrated in the Figures. It will be understood that these terms and those discussed above are intended to encompass different orientations of the device in addition to the orientation depicted in the Figures. The embodiments are not intended to be mutually exclusive so that the features of one embodiment can be combined with other embodiments as long as they do not contradict each other. Terms of degree, such as "substantially", "about" and "approximately" are understood by those skilled in the art to refer to reasonable ranges around and including the given value and ranges outside the given value, for example, general tolerances associated with manufacturing, assembly, and use of the embodiments. The term "substantially" when referring to a structure or characteristic includes the characteristic that is mostly or entirely present in the structure. As used herein, the singular forms "a," "an," and "the" are intended to include the plural forms as well, unless the context clearly indicates otherwise. As used herein, the term "and/or" includes any and all combinations of one or more of the associated listed items. It will be understood that, although the terms first, second, etc., may be used herein to describe various elements, these elements should not be limited by these terms. These terms are only used to distinguish one element from another. For example, a first element could be termed a second element, and, similarly, a second element could be termed a first element without departing from the scope of the present disclosure. For simplification, the parts or elements of one embodiment which are found identically or similarly in the other embodiment will be identified using the same numerical references and will not be described again.

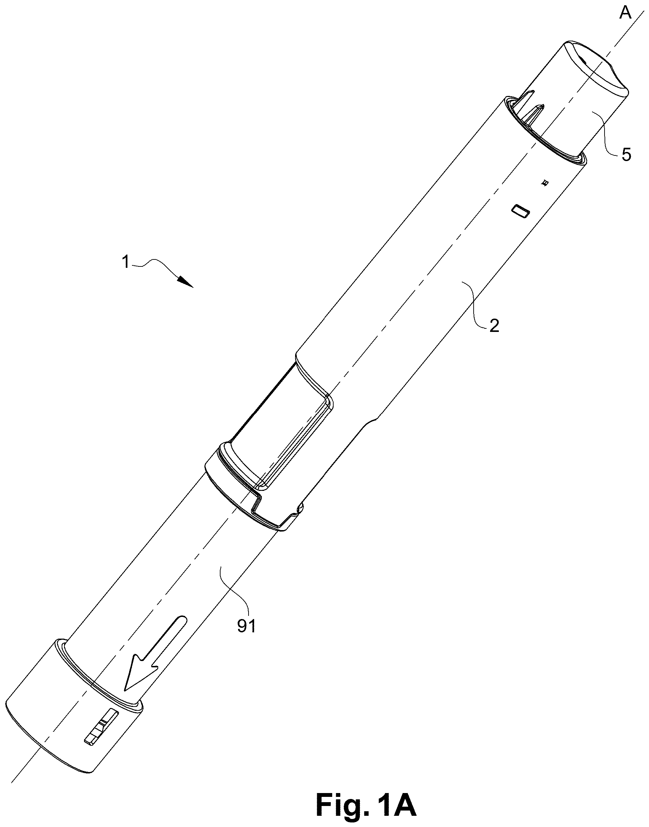

With reference to Figure 1A is shown an autoinjector 1 according to an embodiment of the invention. The autoinjector 1 is configured for automatic injection of a medical product into an injection site. The autoinjector 1 extends along a longitudinal axis A.

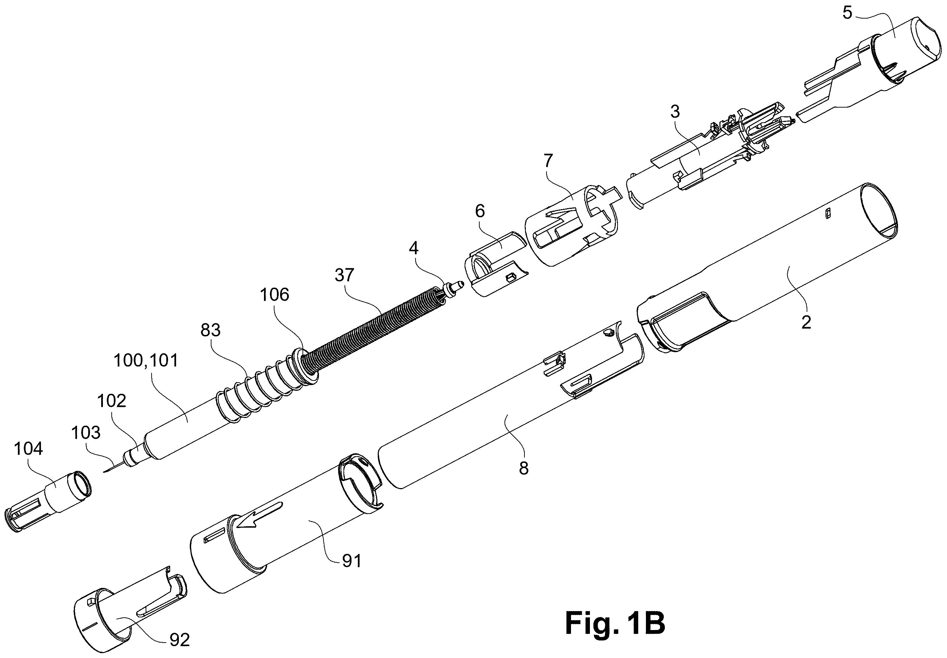

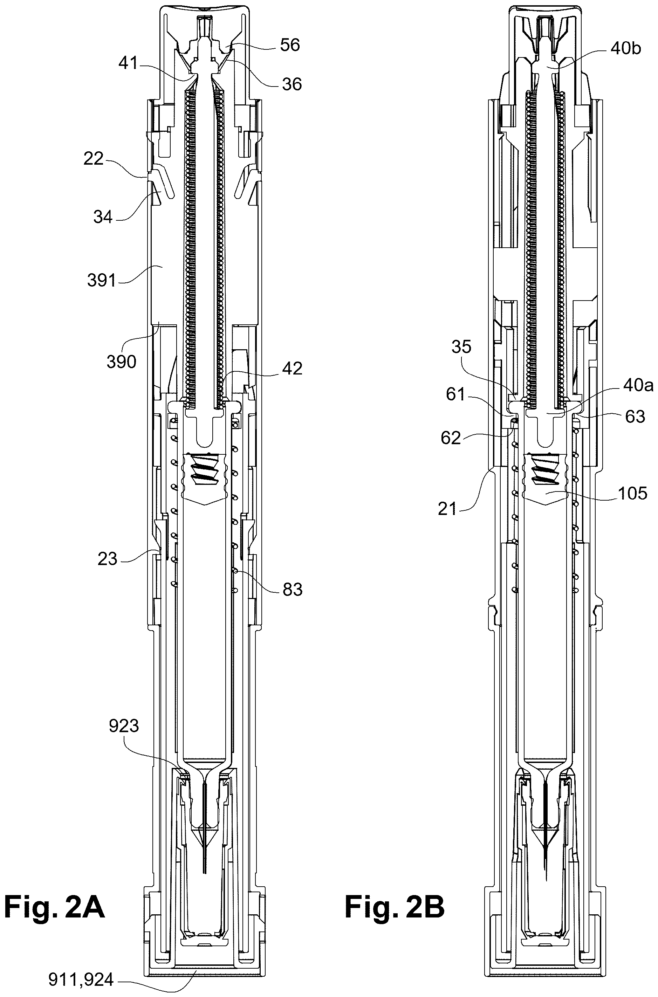

With reference to Figures 1B, 2A, 2B, the autoinjector 1 may include an outer body 2, an inner body 3, an actuation button 5, a holding ring 6, a cam 7, a plunger rod 4, and a needle cover 8. The autoinjector 1 may also include a cap 91 and a remover 92. The injection device may further include a medical container 100, such as a prefillable or prefilled syringe. The medical container 100 may be for instance a 1 mL syringe or a 0,5 mL syringe.

The medical container 100 includes a tubular barrel 101 defining a reservoir configured for containing a medical product. The tubular barrel 101 may include an elongated distal tip 102 defining an axial passageway for passage of the medical product from the reservoir and an injection needle 103 attached to this distal tip 102. Prior to use of the injection device, the injection needle 103 is sealed and protected by a needle shield 104 removably attached to the distal tip 102. A stopper 105, Figure 2B, is arranged within the barrel 101 for pushing the medical product outside the barrel 101 via the distal tip 102 and the injection needle 103. At its proximal end, the barrel 101 may include a radial flange 106. The barrel 101 may be made of a glass or a plastic material.

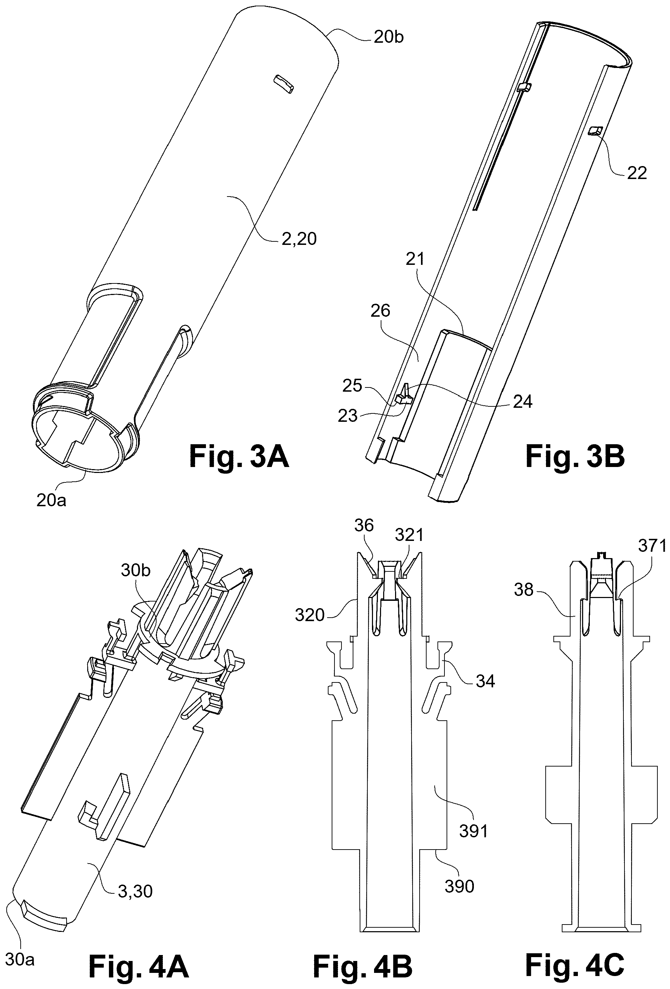

With rerefence to Figures 3A-3B, the outer body 2 is configured for accommodating the medical container 100. The outer body 2 may include a tubular wall 20 defining an inner cavity for receiving the inner body 3, the cam 7, the needle cover 8 and the medical container 100. The tubular wall 20 has an outer surface configured to be grasped by a user. The inner cavity may lead to an opened distal end 20a and an opened proximal end 20b. The outer body 2 may be made of a single piece.

The inner body 3 is configured for retaining the plunger rod 4 in the initial position before injection, and for releasing the plunger rod 4 during the injection process. As illustrated on Figures 4A-4C, the inner body 3 includes an opened distal end 30a, an opened proximal end 30b, and a lateral wall 30 defining an inner cavity for receiving the plunger rod 4 and an injection spring 37. The inner body 3 may be axially and rotationally fixed with respect to the outer body 2. The inner body 3 may be made of a single piece.

It should be contemplated that the inner body 3 is arranged within the outer body 2. That is, the housing of the autoinjector 1 is not made of two parts such as a "lower" or "distal" body and an "upper" or "proximal" body. Instead, the autoinjector 1 of the invention merely has an outer body 2 which contains all the other parts of the autoinjector 1, except for the cap 91 and the remover 92. The cap 91 is directly attached to the outer body 2 which also houses the power-pack formed by the actuation button 5, the inner body 3, the plunger rod 4, and the injection spring 37. The autoinjector 1 does not have a "lower" body. The axial length of the outer body 2 may also be shorter than in the state of the art. The needle cover 8, in the initial position and/or in the retracted position, may mainly extend beyond the distal end of the outer body 2 to which the cap 91 is attached.

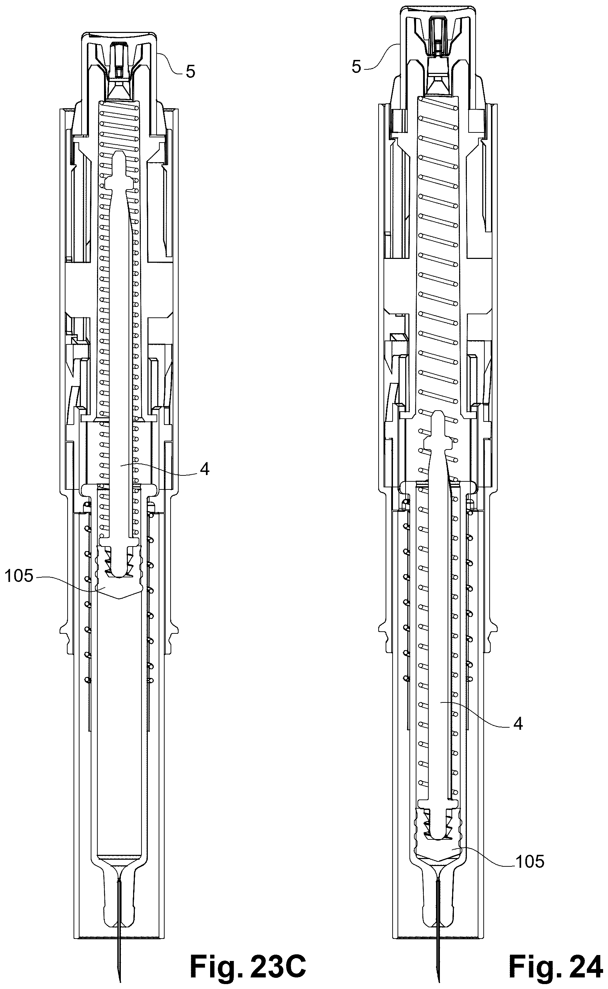

The plunger rod 4 is arranged within the inner body 3 for pushing the stopper 105 in the distal direction in order to first move the medical container 100 together with the holding ring 6 in the distal direction and then move the stopper 105 inside the barrel 101 to expel the medical product contained within the barrel 101. As illustrated in Figures 2A-2B, the plunger rod 4 may be in the form of an elongated rod including a proximal end 40b and a distal end 40a for engaging the stopper 105. The plunger rod 4 is axially movable between an initial position, Figures 2A-2B, and an injection end position, Figure 24, distally located with respect to the initial position, in which the plunger rod 4 presses the stopper 105 against a distal shoulder of the reservoir formed by the barrel 101. Axial movement of the plunger rod 4 from the initial position to the injection end position is due to the injection spring 37 exerting a distal force on the plunger rod 4. The plunger rod 4 may be made of a single piece.

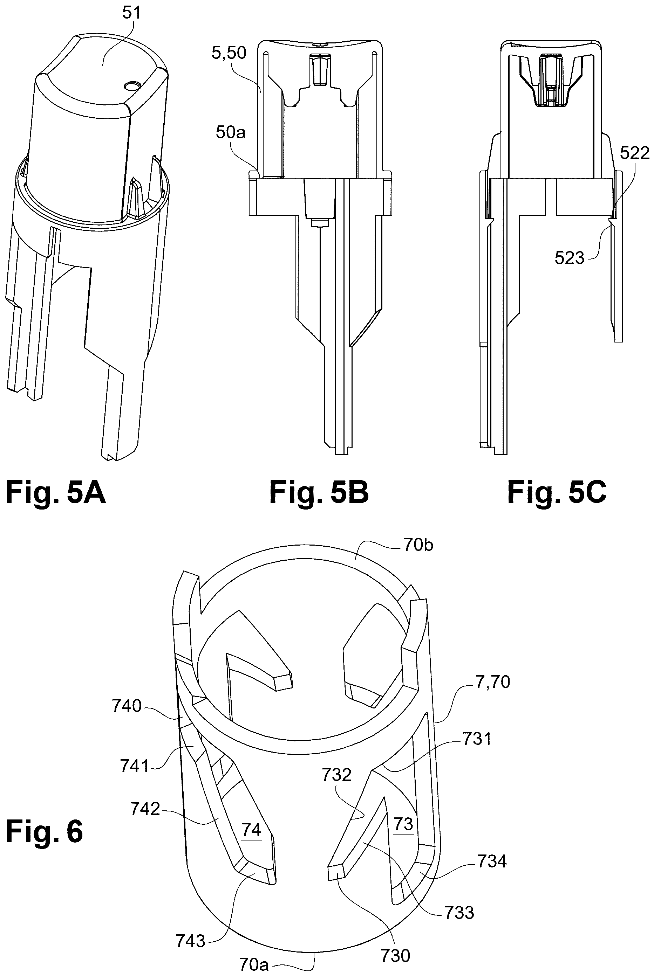

With reference to Figures 5A-5C, the actuation button 5 is configured for releasing the plunger rod 4. The actuation button 5 may be in the form of a push-button and may include a sidewall 50 extending between a closed proximal end, which delimits a pushing surface 51 so that a user's thumb can press against the actuation button 5 in order to move the actuation button 5 in the distal direction, and an opened distal end 50a. The actuation button 5 may be located at the proximal end 20b of the outer body 2 and may at least partially extend outside the outer body 2. The actuation button 5 may be axially movable with respect to the outer body 2 and to the inner body 3 between an initial position, Figures 2A-2B, and an actuation position (not shown), distally located with respect to the initial position in which the actuation button 5 engages the inner body 3 to release the plunger rod 4. Axial movement of the actuation button 5 from the initial position to the actuation position is guided by the inner body 3 and is caused by the user pushing against the actuation button 5 in the distal direction. The actuation button 5 may be rotationally fixed with respect to the outer body 2. The actuation button 5 may be made of a single piece.

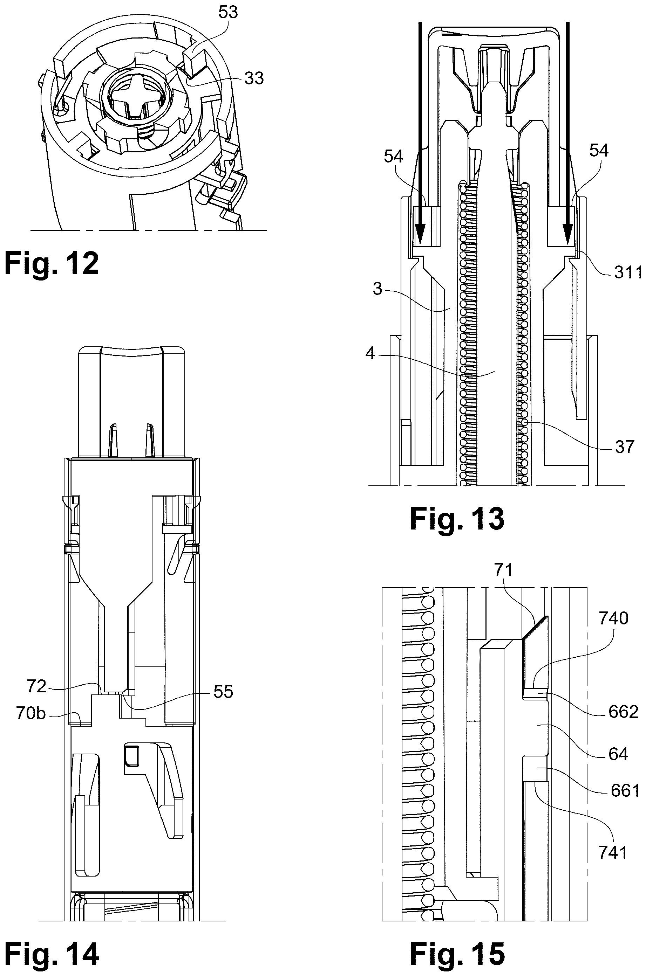

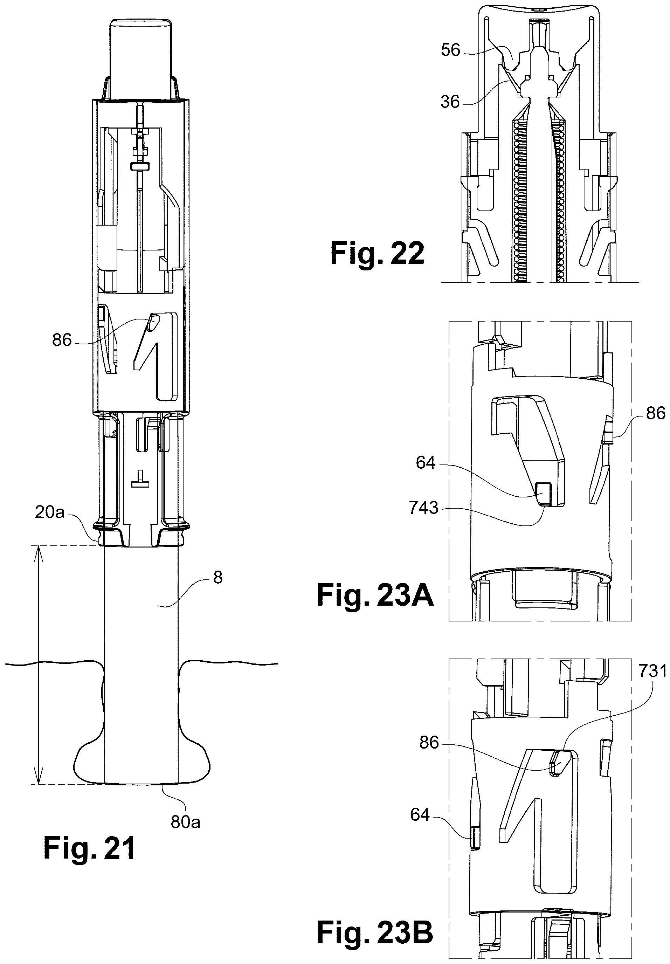

With reference to Figure 6, the cam 7 is arranged within the outer body 2 for controlling steps of an injection operation. The cam 7 may have a cylindrical wall 70, an opened proximal end 70b, and an opened distal end 70a. The cam 7 is rotatably mounted within the outer body 2 and around the longitudinal axis A such that the cam 7 can rotate between a first angular position, Figure 14, a second angular position, Figure 21, and a third angular position, Figure 23B. Rotation of the cam 7 from the first to the second angular position may be caused by retraction of the needle cover 8 in the proximal direction when the autoinjector 1 is pressed against a injection site. Rotation of the cam 7 from the second to the third angular position may be guided by the outer body 2 and may be caused by distal movement of the medical container 100 together with the holding ring 6 after release of the plunger rod 4. The cam 7 may be axially fixed with respect to the outer body 2. The cam 7 may be made of a single piece.

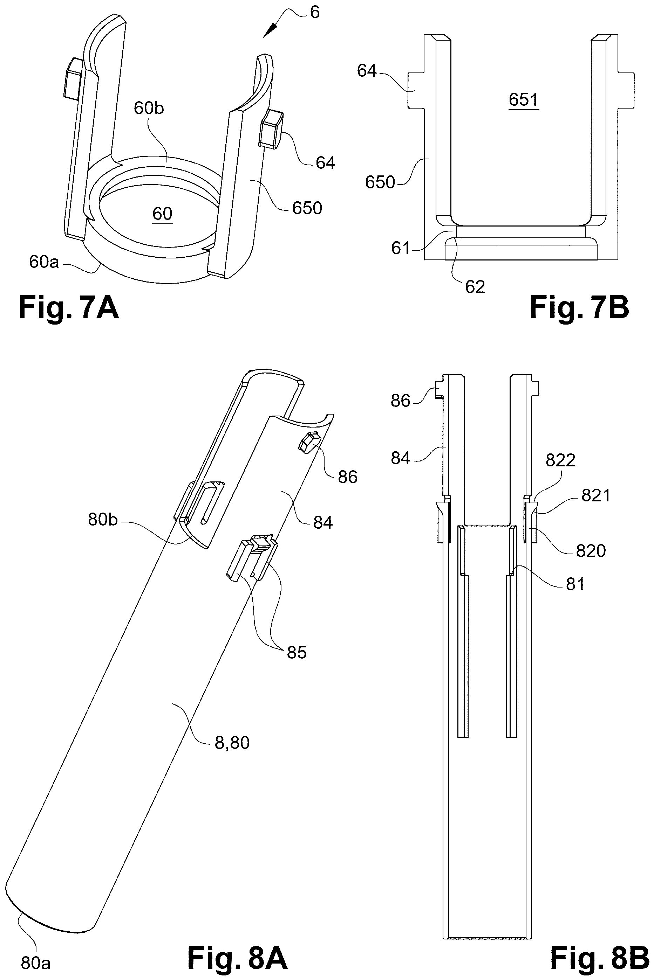

The holding ring 6, is arranged within the cam 7 for supporting the medical container 100. As illsutrated in Figures 7A-7B, the holding ring 6 is in the form of a circumferential ring defining a central opening 60 for receiving the barrel 101 of the medical container 100, a distal side 60a and a proximal side 60b for axially abutting against a distal side of the radial flange 106 of the medical container 100. The holding ring 6 is axially movable with respect to the outer body 2 and along the longitudinal axis A between an initial position, Figure 2A-2B, and a pricking position, Figure 23C, distally located with respect to the initial position. Axial movement of the holding ring 6 may be guided by the needle cover 8 and may be caused by the plunger rod 4 moving in the distal direction under the action of the injection spring 37. The holding ring 6 may be rotationally fixed with respect to the outer body 2. The holding ring 6 may be made of a single piece.

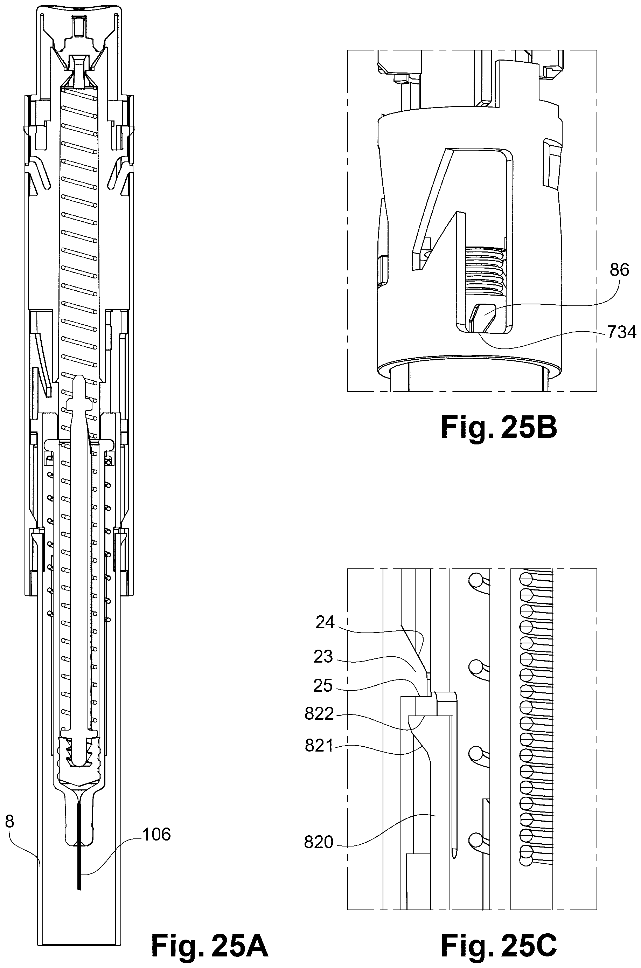

With reference to Figures 8A-8B, the needle cover 8 is arranged within the outer body 2 for protecting the user against needle stick injuries. The needle cover 8 includes an opened distal end 80a, an opened proximal end 80b and a lateral wall 80 defining an inner cavity for receiving the medical container 100. The needle cover 8 may be axially movable between an initial position, Figures 2A-2B, a retracted position, Figure 21, proximally located with respect to the initial position, and a safety position, Figures 25A-25C, distally located with respect to the retracted position. Axial movement of the needle cover 8 may be guided by the outer body 2. Movement of the needle cover 8 from the initial position to the retracted position is caused by the user pressing the distal end 80a of the needle cover 8 against the injection site. Movement of the needle cover 8 from the retracted position to the safety position is caused by a safety spring 83. The needle cover 8 may be rotationally fixed with respect to the outer body 2. The needle cover 8 may be made of a single piece. It is contemplated that the axial dimension of the portion of the needle cover 8 that extends outside the outer body 2 in the retracted position may be equal to or greater than that of the portion of the needle cover 8 that is inside the outer body 2. For instance, the portion of the needle cover 8 that extends beyond the distal end 20a of the outer body 2 may be equal to or greater than 35 mm, preferably equal to or greater than 40 mm, and even more preferably equal to or greater than 41,5 mm.

The safety spring 83 may be a coil spring including a distal end leaning against a proximal seat 81, Figure 8B, defined by inner axial ribs of the needle cover 8, and a proximal end leaning against a distal seat 62 delimited at a distal side of an inner flange 61 of the holding ring 6, Figures 2B and 7B.

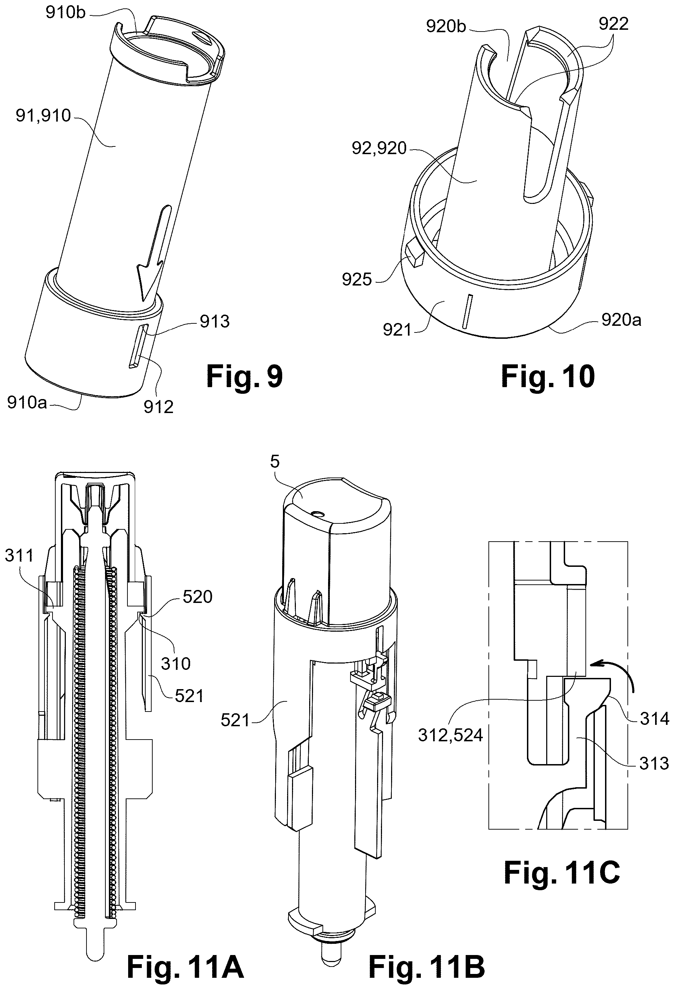

The cap 91 may be arranged at the distal end of the outer body 2 for protecting the needle cover 8 and the medical container 100 before use of the autoinjector 1. Specifically, the cap 91 protects against inadvertent actuation of the autoinjector 1 during a drop test or any accidental drop of the autoinjector 1. The cap 91 extends around the needle cover 8. As illustrated in Figure 9, the cap 91 may include an opened proximal end 910b, an opened distal end 910a, and a tubular lateral wall 910. The cap 91 is removably attached to the outer body 2. The cap 91 may be made of a single piece. The cap 91 is substantially longer than caps of the state of the art. Specifically, the cap 91 extends over the entire needle cover 8. As a result, when the cap 91 is detached, there is a great distance between the distal end 80a of the needle cover 8 and the distal end 20a of the housing formed by the outer body 2. This prevents pinching of the patient's skin or avoids any skin and outer body 2 interactions that could potentially increase the effort to fully press the needle cover 8.

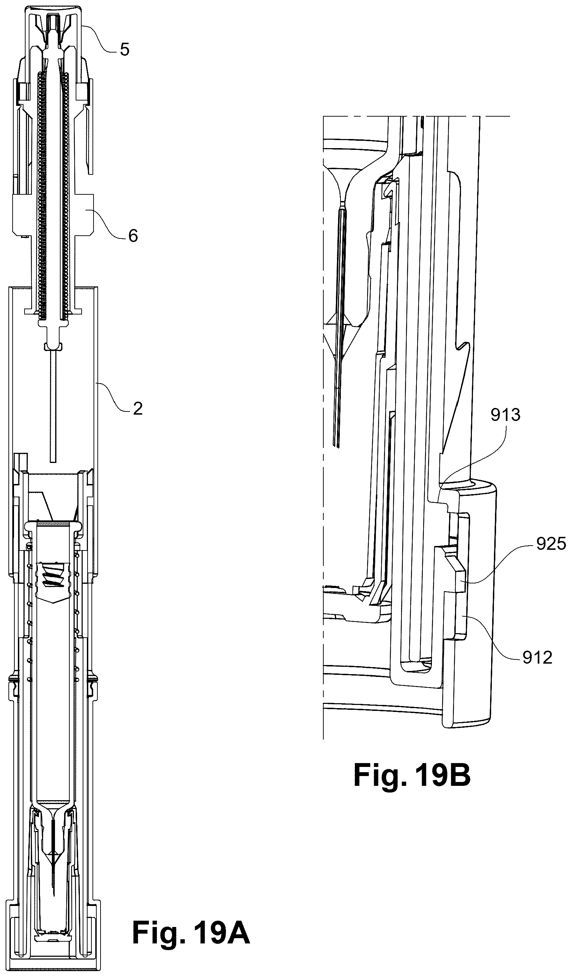

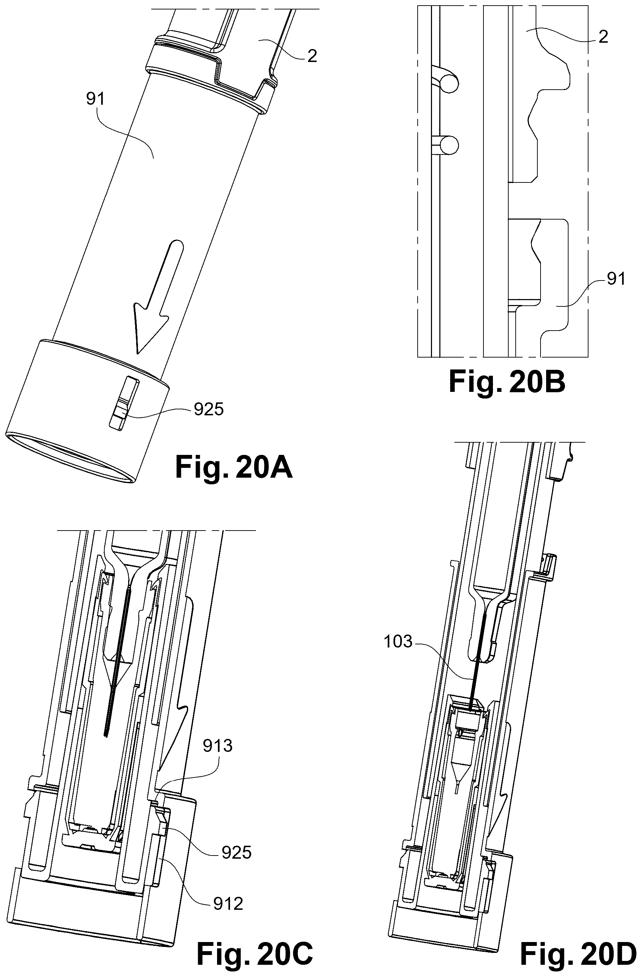

With reference to Figures 2A-2B and 10, the remover 92 is arranged within the cap 91 for removing the needle shield 104 from the medical container 100, thereby exposing the injection needle 103, when the cap 91 is detached from the outer body 2. The remover 92 may include an inner sleeve 920 extending between an opened proximal end 920b and an opened distal end 920a, and an outer sleeve 921 surrounding the inner sleeve 920. The remover 92 and the cap 91 are axially movable with respect to one another along the longitudinal axis A between an initial position, Figure 19B, in which the cap 91 can be detached from the outer body 2 without exerting a distal force on the remover 92, and a removal position, Figure 20C, in which the cap 91 is detached from the outer body 2 and axially abuts against the remover 92. Relative axial movement between the cap 91 and the remover 92 is caused when the cap 91 is pulled off the outer body 2. The remover 92 may be made of a single piece.

With reference to Figures 11A-11C, the autoinjector 1 includes snap-fit members 310, 520 configured for allowing assembly of the actuation button 5 to the inner body 3. One of the snap-fit member 520, which may be arranged on a distally protruding leg 521 of the actuation button 5, may be in the form of an inward protrusion delimiting a proximal abutment 522, Figure 5C, and a distal ramp 523. The other snap-fit member 310 may be a distal side of a functional flange 311 of the inner body 3. The snap-fit members 310, 520 block the actuation button 5 relative to the inner body 3 in the proximal direction.

Besides, Figure 11C, a distal blocking surface 524 may be arranged at the distal end 50a of the actuation button 5 for axially abutting against a provisional proximal blocking surface 312 arranged at a free proximal end of a T-shaped proximally protruding resilient leg 313 of the inner body 3. A camming surface 314 may be provided on an outward protrusion of this resilient leg for radially abutting against the outer body 2 when the outer body 2 is assembled to the inner body 3. That is, the resilient leg 313 is radially movable between a blocking position, Figures 11B-11C, in which the actuation button 5 is prevented from moving distally with respect to the inner body 3, and a release position (not shown), in which the proximal blocking surface 312 of the resilient leg 313 is shifted away such that the actuation button 5 can be moved in the distal direction with respect to the inner body 3. The proximal and distal blocking surfaces 312, 524 thus form provisional securing means for axially securing the actuation button 5 with respect to the inner body 3 before assembly of the outer body 2 to the inner body 3.

As illustrated in Figure 12, the actuation button 5 may include an indexer 53, which may be in the form of an axial rib running along an inner surface of the sidewall 50 of the actuation button 5, this indexer 53 being configured for complementarily engaging an indexer 33 arranged on the inner body 3. The indexer 33 of the inner body 3 may be a notch provided on the functional flange 311.

As illustrated in Figure 13, the actuation button 5 may include axial push openings 54 axially facing the inner body 3 and arranged through the actuation button 5 for allowing passage of fingers of an assembly machine so that the fingers of the assembly machine can distally push against a proximal side of the functional flange 311 of the inner body 3 in order to push the so-called power pack, i.e. the assembly formed by the actuation button 5, the inner body 3, the plunger rod 4 and the injection spring 37, inside the outer body 2.

With reference to Figure 14, the actuation button 5 and the cam 7 may include axial blocking members 55, 72 formed by a distal blocking surface 55 and a proximal blocking surface configured for axially abutting against each other when the cam 7 is in its first angular position, so that the actuation button 5 is prevented from axially moving towards the actuation position before retraction of the needle cover 8. The axial blocking member 55 of the actuation button 5 may be arranged at a distal end of the distally protruding legs 521. The axial blocking member 72 of the cam 7 may extend at a proximal end of a tab proximally protruding from the proximal end 70b of the cam 7.

As illustrated in Figure 2A, the actuation button 5 further includes a deflector 56, which may distally protrude from the closed proximal end 50b of the actuation button 5, for abutting against a camming surface 36 of the inner body 3 in order to release the plunger rod 4 when the actuation button 5 is pressed to the actuation position.

The inner body 3 includes a retainer which may be in the form of two diametrically opposite proximal legs 320 arranged at the proximal end 30b of the inner body 3 for provisionally retaining the plunger rod 4 in the initial position. The retainer has a proximal stop 321 for axially abutting against a distal stop 41 of the plunger rod 4, Figure 2A, and the camming surface 36 for causing radial outward movement of the retainer when the actuation button 5 is pressed to the actuation position. The radial outward movement of the retainer allows for moving the proximal stop 321 out of the path of the plunger rod 4.

As illustrated in Figure 2A, the inner body 3 includes a snap-fit member, such as a resilient leg 34 having a radial protrusion for engaging a complementary shaped hole 22 provided through the tubular wall 20 of the outer body 2, for axially and rotationally securing the inner body 3 to the outer body 2. The hole 22 may also help disassemble the inner body 3 and the outer body 2 by radially inwardly pressing the resilient leg 34.

Still with reference to Figure 2A, the inner body 3 may include an anti-wobbling feature 35, such as a distal stop which may delimited by two radials tabs arranged at the distal end 30a of the inner body 3, for preventing small axial movements of the medical container 100. This prevents the medical container 100 from generating a rattling noise for instance during transport or use of the autoinjector 1. Indeed, the anti-wobbling feature 35 maintains the radial flange 106 of the medical container 100 against a corresponding anti-wobbling feature 63 of the holding ring 6 in the form of a proximal abutment delimited by a proximal side of the inner flange 61 of the holding ring 6.

The inner body 3 may have a distal seat 371, for instance arranged at two diametrically opposite axial legs 38 axially protruding from the functional flange 311 of the inner body 3, for providing support to a proximal end of the injection spring 37. The opposite distal end of the injection spring 37, which may a coil spring extending around the plunger rod 4 and inside the cavity delimited by the lateral wall of the inner body 3, may lean against a proximal seat 42, Figure 2A, defined by a proximal side of an outer flange located at the distal end of the plunger rod 4.

Turning now to Figures 6 and 21, 23A-23B, 25B, the cam 7, the holding ring 6 and the needle cover 8 include sequencing features 64, 73, 74, 86 configured for sequencing use steps of the autoinjector 1. The cam 7 includes a first sequencing feature in the form of a first track 73 engaged by the sequencing feature 86 of the needle cover 8, and a second sequencing feature in the form of a second track 74 engaged by the sequencing feature 64 of the holding ring 6. The sequencing feature 86 of the needle cover 8 may be in the form of a radial outer protrusion extending from a proximal leg 84 of the needle cover 8. The sequencing feature 64 of the holding ring 6 may also be in the form of a radial outer protrusion extending from a proximal leg 650 of the holding ring 6.

The first track 73 may include a first proximal stop 730, a distal stop 731, an upper camming surface 732, a lower camming surface 733, and a second proximal stop 734. The first proximal stop 730 may axially abut against the sequencing feature 86 of the needle cover 8 when the needle cover 8 is in the initial position. The distal stop 731 may axially abut against the sequencing feature 86 of the needle cover 8 when the needle cover 8 is in the retracted position. The second proximal stop 734 may axially abut against the sequencing feature 86 of the needle cover 8 when the needle cover 8 is in the safety position. The upper camming surface 732 causes rotation of the cam 7 from the first to the second angular position by abutment against the sequencing feature 86 of the needle cover 8 when the needle cover 8 moves from the initial to the retracted position. The lower camming surface 733 allows the cam 7 to rotate back from the second to the first angular position should a user eventually decides to move the autoinjector 1 out of contact from the injection site (before pressing the actuation button 5), thereby causing the needle cover 8 to move back from the retracted position to the initial position, for example to re-position the autoinjector 1 on another and more appropriate injection site.

The second track 74 may include a distal stop 740 for axially abutting against the sequencing feature 64 of the holding ring 6 in order to block distal movement of the medical container 100 before retraction of the needle cover 8, a first proximal stop 741 for limiting proximal movement of the medical container 100, a camming surface 742 for causing rotation of the cam 7 from the second to the third angular position by abutment against the sequencing feature 64 of the holding ring 6, and a second proximal stop 743 for stopping distal movement of the medical container 100, thereby defining the injection depth.

It is contemplated that, in an initial position of the medical container 100, a distal gap 661 and a proximal gap 662 may exist between the sequencing feature 64 of the holding ring 6 and, respectively, the first proximal stop 741 and the distal stop 740 of the second sequencing feature 74 of the cam 7.

The cam 7 is axially secured with respect to the outer body 2 by a distal blocking surface 390, Figure 2A, which may be arranged at a proximal end of radial plates 391 of the inner body 3, configured for axially abutting against the proximal end 70b of the cam 7, and by a proximal blocking surface 21, Figure 2B, which may be arranged at a proximal shoulder of the outer body 2.

As illustrated in Figure 15, a chamfer 71 may be provided at the proximal end 70b, or the distal end 70a, of the cam 7 in order to ease insertion of the needle cover 8.

With reference to Figures 7A-7B and 8A-8B, the holding ring 6 and the needle cover 8 may include guiding members 650, 651, 84 configured for guiding axial movement of the holding ring 6 along the longitudinal axis A. A first guiding member may be formed by the proximal legs 84 of the needle cover 8 which may be shaped to complementarily engage notches 651 of the holding ring 6. These notches 651, delimited between proximal legs 650 of the holding ring 6, may thus form a second guiding member. Since the guiding members 84, 650 orthoradially abut against each other, relative rotation between the holding ring 6 and the needle cover 8 is prevented. It is contemplated that the proximal legs 650 of the holding ring 6 and the proximal legs 84 of the needle cover 8 may form a cylinder. That is, the proximal legs 650 of the holding ring 6 and the proximal legs 84 of the needle cover 8 are aligned in a circumferential direction. Therefore, having a holding ring 6 guided by the needle cover 8 enables to reduce the overall diameter of the autoinjector 1. The autoinjector 1 is compact in a radial dimension. The needle cover 8 may include two diametrically opposite proximal legs 84, and the holding ring 6 may include two complementarily shaped and diametrically opposite proximal legs 650, although embodiments are not limited thereto.

The needle cover 8 and the outer body 2 include guiding members 26, 85 configured for guiding axial movement of the needle cover 8 with respect to the outer body 2. The guiding members 26, 85 may also be configured for preventing relative rotation between the needle cover 8 and the outer body 2. For instance, as illustrated in Figures 3B and 8A, a guiding member may be in the form of an axial groove 26 arranged on an inner surface of the lateral wall 20 of the outer body 2, and another guiding member 85 may be in the form of one or more axial ribs 85 arranged on an outer surface of the lateral wall 80 of the needle cover 8.

The autoinjector 1 further includes locking elements for locking the needle cover 8 in the safety position after injection, in order to protect the user against needle stick injuries. With reference to Figure 25C, the locking elements may include a snap-fit member, such as a resilient leg 820 which may be arranged on the needle cover 8, and a radial inward protrusion 23 which may be arranged on the inner surface of the lateral wall 20 of the outer body 2. The resilient leg 820 of the needle cover 8 is radially deformable and includes a distal ramp surface 821 configured to abut against a proximal ramp surface 24 of the protrusion 23 for easing passage of the resilient leg over this protrusion 23 when the needle cover 8 distally moves toward the safety position. The resilient leg 820 further includes a proximal blocking surface 822 configured for axially abutting against a distal blocking surface 25 of the protrusion 23 of the outer body 2 when the needle cover 8 is in the safety position, such that the needle cover 8 cannot move back in the proximal direction.

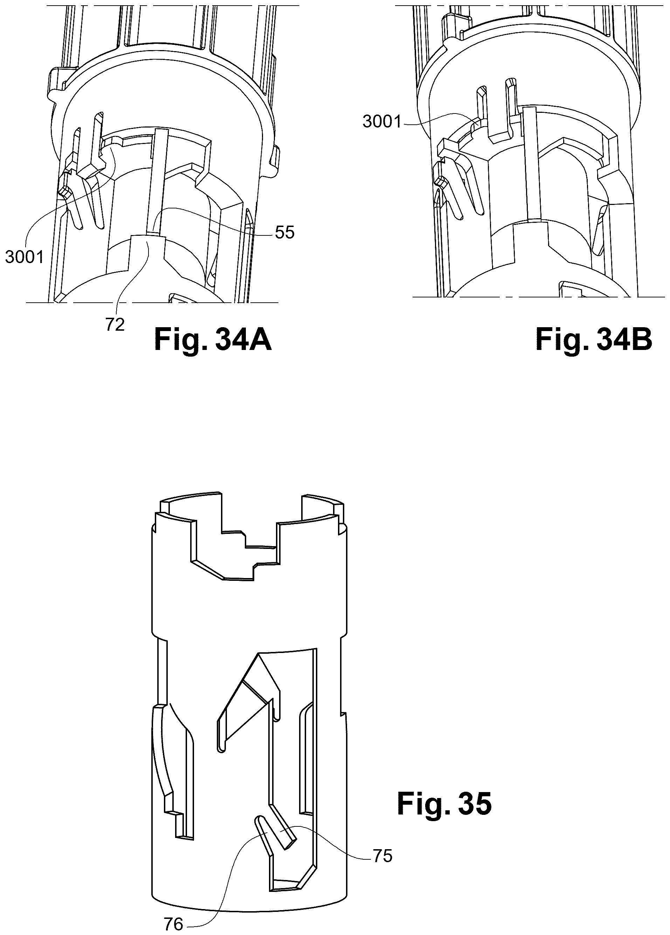

In an alternative embodiment illustrated in Figure 35, the locking elements may instead include first track 73 of the cam 7 having a resilient tab 75 extending on the path of the sequencing feature 86 of the needle cover 8 when the needle cover 8 moves from the retracted to the safety position. The resilient tab 75 acts as a ratchet for capturing the sequencing feature 86 at a distal end of the first track 73. The resilient tab 75 flexes for allowing passage of the needle cover 8 in the distal direction, but prevents the needle cover 8 from moving back in the proximal direction. An oblique slot 76 may extend alongside the resilient tab 75 to trap the sequencing feature 86 of the needle cover 8. This may help reduce the axial length of the outer body 2.

As visible in Figure 2A, the cap 91 includes a distal opening 911 for allowing a push-back step. The push-back step occurs after insertion of the medical container 100 within the outer body 2, for ensuring that the remover 92 does engage the needle shield 104. To that end, after insertion of the medical container 100 in the distal direction, the medical container 100 is slightly pushed back in the proximal direction such that the remover 92 is engaged with the needle shield 104. As a result, the remover 92 will reliably pull the needle shield 104 when the cap 91 will be detached from the outer body 2. It is contemplated that the remover 92 also includes a distal opening 924, aligned with the distal opening 911 of the cap 91, for allowing the push-back step.

Besides, the cap 91 and the remover 92 include sequencing features 912, 913, 925 configured for allowing a two-step removal of the cap 91 and the needle shield 104. That is, the cap 91 is first detached from the outer body 2 and, after detachment of the cap 91, the needle shield 104 is then removed from the medical container 100 by the remover 92. With reference to Figure 9, the sequencing features of the cap 91 may include an axial slot 912 allowing arranged through the tubular lateral wall 910 of the cap 91. The axial slot 912 is engaged by the sequencing feature, such as an outer protrusion 925 arranged on the outer sleeve 921, of the remover 92. The axial slot 912 of the cap 91 and the outer protrusion 925 of the remover 92 thus allow for axial movement of the cap 91 with respect to the remover 92 to some extent, and may prevent relative rotation between the cap 91 and the remover 92. The sequencing features of the cap 91 also include a distal abutment 913, in the form of a distal shoulder, configured for axially abutting against the remover 92, and more specifically against a proximal end of the outer sleeve 921, after the cap 91 gets detached from the outer body 2. Therefore, the distal abutment 913 enables to pull the remover 92 together with the cap 91 in the distal direction after detachment of the cap 91. The two-step removal of the cap 91 and needle shield 104 reduces the force that needs to be exerted by the user in comparison with a situation where the user would have to simultaneously remove the cap 91 from the outer body 2 and the needle shield 104 from the medical container 100.

The remover 92 includes hooks 922 arranged at a proximal end of the inner sleeve 920 for engaging an axial gap 923, Figure 2A, between a distal shoulder of the barrel 101 and a proximal end of the needle shield 104, such that the hooks 922 can axially abut against the proximal end of the needle shield 104 when the cap 91 is pulled in the distal direction.

The autoinjector 1 of the invention is thus compact while still permitting to safely inject a medical product into an injection site. The short axial length and reduced diameter of the autoinjector 1 makes it less cumbersome and easier to handle the autoinjector 1 of the invention. Due to ist compact size, less raw material is required for manufacturing the autoinjector 1. This results in an improved sustainability. Risks of pinching the user's skin between the outer body 2 and the needle cover 8, or any skin and outer body interactions that could potentially increase the effort to fully press the needle cover, are limited. Besides, the actuation button 5 does not require the user to press the whole autoinjector 1 against the injection site. This accordingly limits the force needed to apply the autoinjector 1 against the injection site, and makes the autoinjector 1 of the invention easier to use.

Assembly of the autoinjector 1 is described below.

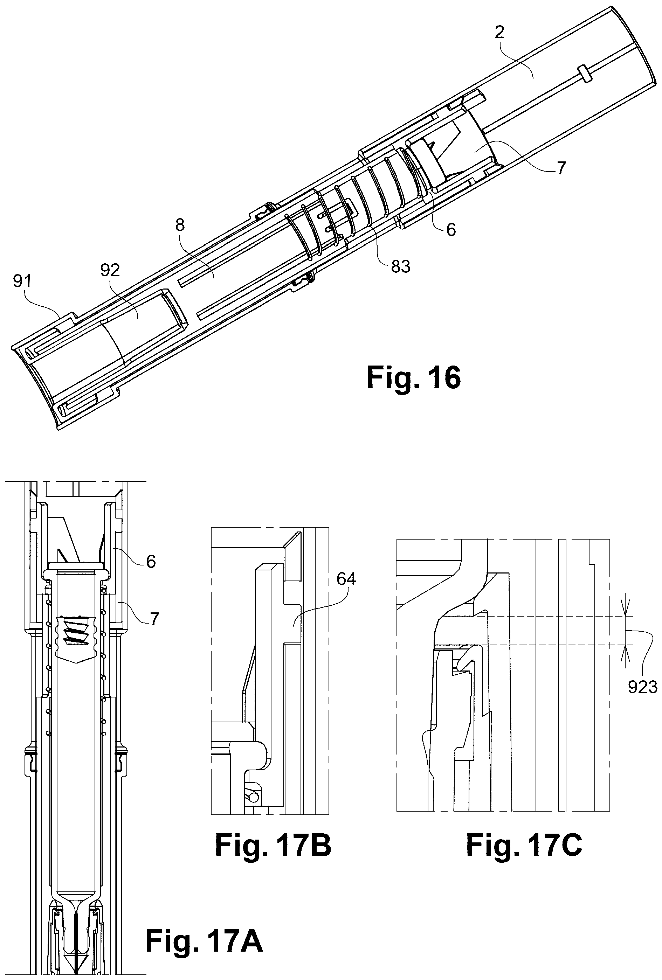

The cam 7 may be snap-fitted to the needle cover 8, by inserting the sequencing feature 86 of the needle cover 8 into the first track 73 of the cam 7. The safety spring 83 may be positioned in the needle cover 8. The holding ring 6 may then be snap-fitted in the cam 7, by engaging the sequencing feature 64 of the holding ring 6 into the second track 74 of the cam 7. The safety spring 83 thus gets sandwiched between the needle cover 8 and the holding ring 6. The assembly formed by the needle cover 8, the cam 7, the safety spring 83 and the holding ring 6 is then inserted into the outer body 2. The cap 91, including the remover 92, may be attached to the distal end 20a of the outer body 2. At this stage, the autoinjector 1 is as shown in Figure 16.

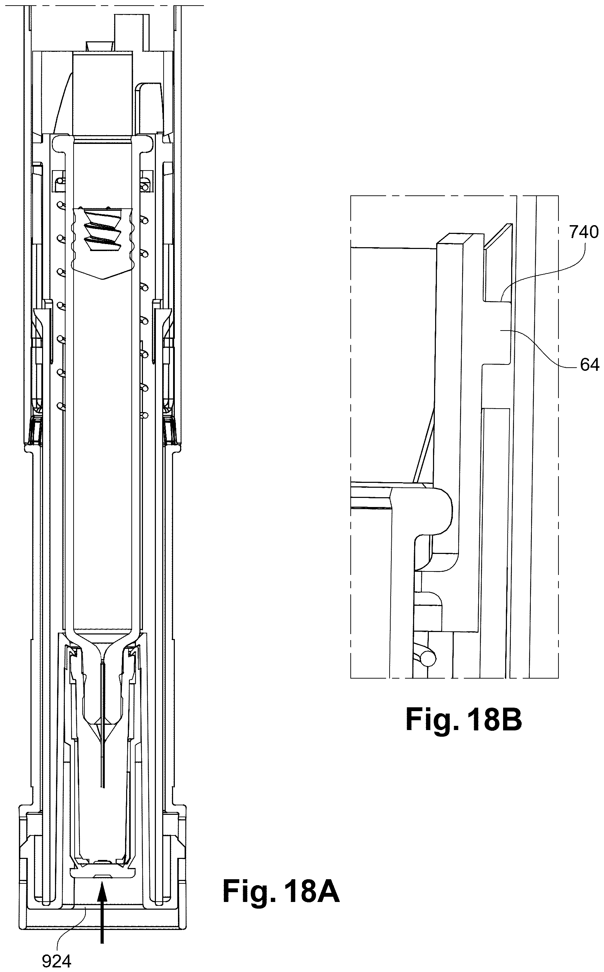

The medical container 100 may be inserted inside the outer body 2 and the needle cover 8, Figure 17A. The radial flange 106 abuts against the holding ring 6, which in turn abuts against the cam 7, figure 17B. The axial stroke between the distal stop 740 and the first proximal stop 741 of the cam 7 allows for pushing the medical container 100 in the distal direction such that the hooks of the remover 92 pass beyond the proximal end of the needle shield 104, Figure 17C. In a subsequent stage, Figure 18A, a push-back step enables to push against the distal end of the needle shield 104 to move the medical container 100 in the proximal direction such that the hooks engage the axial gap 923. The push-back step further causes the holding ring 6 to abut against the distal stop 740 of the cam 7 under the force of the safety spring 83, Figure 18B, and may also cause the medical container 100 to move out of contact from the inner flange 61 of the holding ring 6.

With reference to Figure 19A, the power-pack is inserted in the outer body 2. This insertion causes the resilient leg 313 of the inner body 3 to radially inwardly deflect when abutting against the inner surface of the tubular wall 20 of the outer body 2, such that the cap 91 is no longer blocked in the distal direction. Besides, the snap-fit members 310 of the inner body 3 engage the holes 22 of the outer body 2, such that the inner body 3 is axially and rotationally secured to the outer body 2. Also, the inner body 3 presses the radial flange 106 of the medical container 100 against the inner flange 61 of the holding ring 6. The holding ring 6 reaches a position such that the sequencing feature 64 is distant from the distal stop 740 and from the first proximal stop 741 of the cam 7, Figure 15. During distal movement of the medical container 100 due to the insertion of the inner body 3, the remover 92 can slide relative to the cap 91, Figure 19B, and the sequencing feature 925 of the remover 92 is axially distant from the distal abutment of the cap 91.

Use of the autoinjector 1 will be described below.

With reference to Figure 20A, the cap 91 is detached from the outer body 2. The remover 92 firstly stays fixed with respect to the medical container 100. After separation of the cap 91 from the outer body 2, Figure 20B, the distal abutment 913 of the cap 91 abuts against the remover 92, Figure 20C. Further distal movement of the cap 91 causes the remover 92 to pull the needle shield 104 in the distal direction, so that the needle shield 104 gets removed from the medical container 100, Figure 20D.

The user then presses the distal end 80a of the needle cover 8 8 against the injection site. Due to the absence of a "lower" body, and thus due to the high distance between the distal end of the needle cover 8 and the distal end 20a of the outer body 2, risks that the patient's skin gets pinched between the needle cover 8 and the housing, or any skin and outer body interactions that could potentially increase the effort to fully press the needle cover, are limited. The needle cover 8 moves to the retracted position, Figure 21. This causes the cam 7 to rotate from the first angular position to the second angular position. As a result, the axial blocking member 72 of the cam 7 gets out of the way of the actuation button 5: the user can now press the actuation button 5 in the distal direction to trigger automatic pricking and then injection.

By moving the actuation button 5 in the distal direction, the user causes the deflector 56 of the actuation button 5 to abut against the camming surface 36 of the retainer of the inner body 3. The proximal legs 320 radially outwardly deflect, thereby releasing the plunger rod 4. Under the pressure of the injection spring 37, the plunger rod 4 thus moves in the distal direction. Due to the friction between the stopper 105 and the barrel 101, this first causes the medical container 100 to move in the distal direction. The injection needle 103 penetrates into the injection site. The distal movement of the medical container 100 also causes the holding ring 6 to move distally, so that the sequencing feature 64 of the holding ring 6 slides against the camming surface 742 of the cam: the cam 7 rotates from the second angular position to the third angular position, Figures 23A, 23B. The second proximal stop 743 of the cam 7 stops distal movement of the holding ring 6 and of the medical container 100, Figures 23A, 23C. Injection can begin.

The injection spring 37 goes on exerting a distal force on the plunger rod 4, which accordingly distally moves the stopper 105 inside the barrel 101, thereby expelling the medical product into the injection via the injection needle 103. Injection stops when the stopper 105 reaches the distal end of the reservoir formed by the barrel 101, Figure 24.

The user removes the autoinjector 1 from the injection site. Under the pressure of the safety spring 83, the needle cover 8 distally moves from the retracted position to the safety position, Figure 25A, until the sequencing feature 86 of the needle cover 8 abuts against the second proximal stop 734 of the cam 7, Figure 25B. Meanwhile, the resilient leg 820 of the needle cover 8 has passed over the protrusion 23 of the outer body 2, such that the needle cover 8 is prevented from moving back in the proximal direction, Figure 25C.

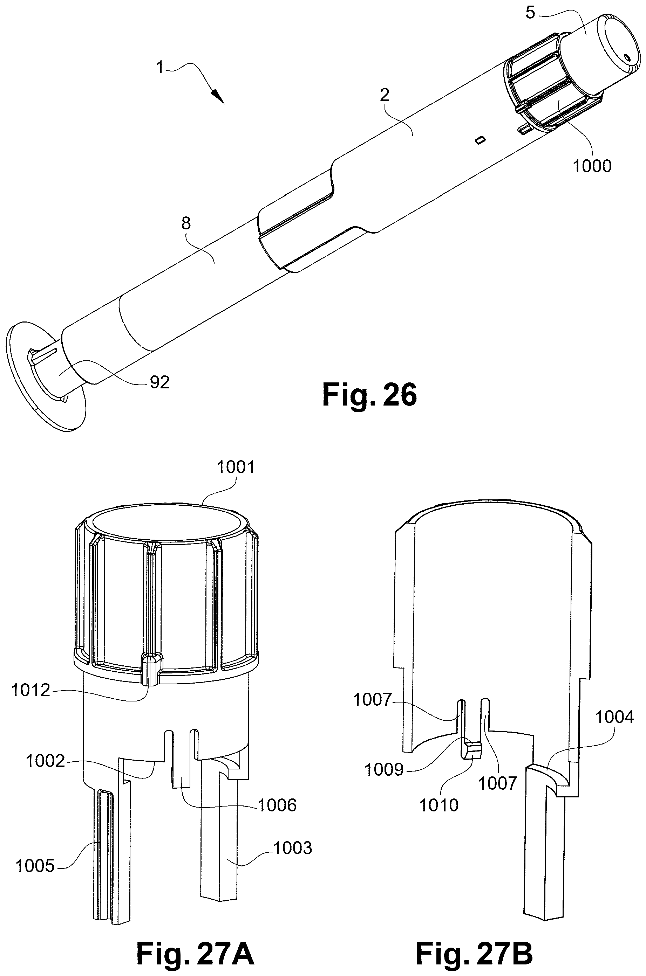

Figures 26 to 34B illustrate the autoinjector 1 according to another embodiment of the invention. In this embodiment, the autoinjector 1 has no cap 91 and no push-back step is required. Besides, the autoinjector 1 here includes a locker 1000. For simplification, the parts or elements of one embodiment which are found identically or similarly in the other embodiment will be identified using the same numerical references and will not be described again. It is reminded that the embodiments are not intended to be mutually exclusive so that the features of one embodiment can be combined with other embodiments as long as they do not contradict each other.

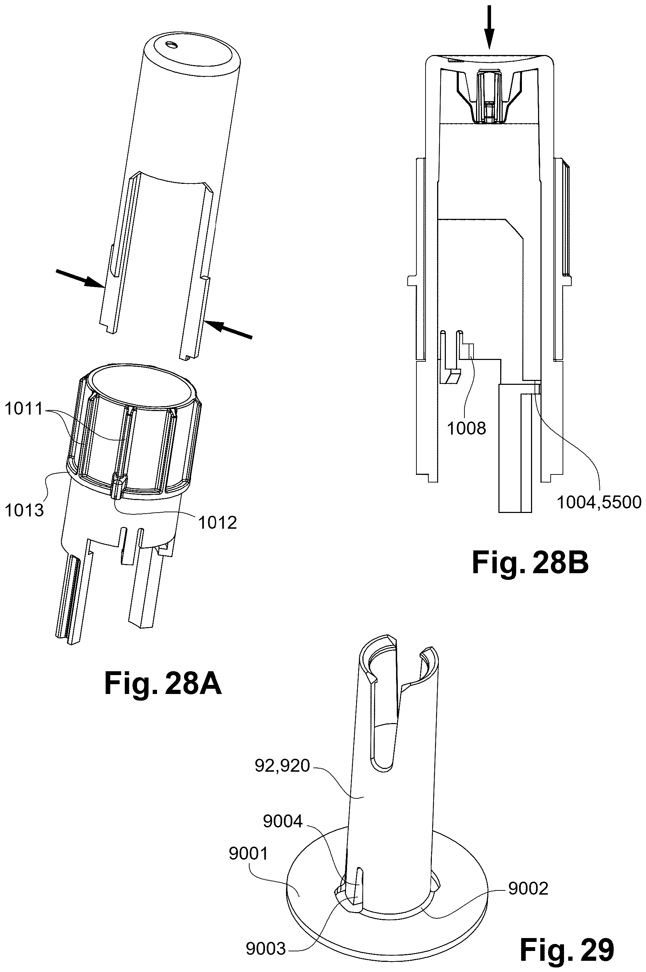

With reference to Figures 27A-27B, the locker 1000 is arranged at the proximal end 20b of the outer body 2 and is configured for axially blocking the actuation button 5 and for axially blocking the needle cover 8 before use of the autoinjector 1. The locker 1000 may be in the form of a cylindrical ring including an opened proximal end 1001 and an opened distal end 1002. Two diametrically opposite distal legs 1003 may axially extend from the distal end 1002 of the locker 1000. The distal legs 1003 may define a proximal shoulder 1004 and may include an axial slot between two axial ribs 1005. Besides, a resilient leg 1006 may distally extend between two notches 1007 at the distal end of the locker 1000. The resilient leg 1006 has a hook defining a proximal abutment 1009 and a distal ramp 1010. A proximal stop 1008, Figure 28B, may be delimited by a radial protrusion ajacent to the notches. Axial ribs 1011 may be regularly distributed in a circumferential direction on an outer surface of the locker 1000 for easing handling of the locker 1000 by the user. An outer protrusion 1012 may be arranged on said outer surface, and may protrude from one of said axial ribs 1011. The locker 1000 has a distal shoulder 1013 separating a proximal portion, which is designed to extend outside the outer body 2, and a distal portion, which designed to extend within the outer body 2.

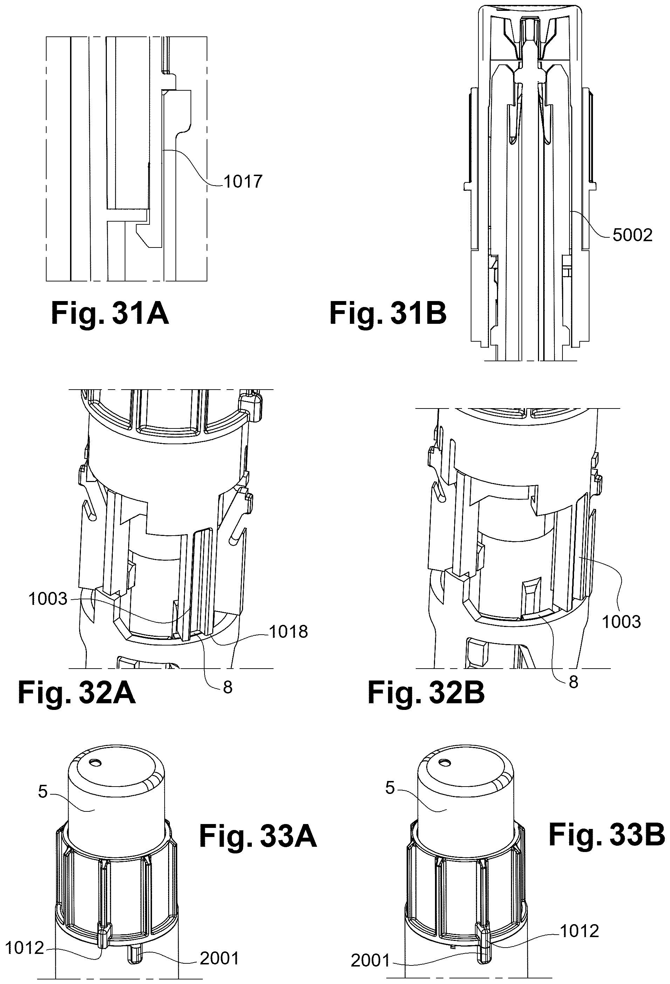

The locker 1000 is rotationally movable with respect to the outer body 2 and the actuation button 5, and around the longitudinal axis A, between a locking position, Figure 33A, in which the locker 1000 prevents proximal movement of the needle cover 8 and prevents distal movement of the actuation button 5, and a release position, Figure 33B, in which the locker 1000 allows for proximal movement of the needle cover 8 and distal movement of the actuation button 5. Rotation of the locker 1000 from the locking to the release position is caused by the user. The locker 1000 may be axially fixed with respect to the outer body 2. The locker 1000 may be made of a single piece.

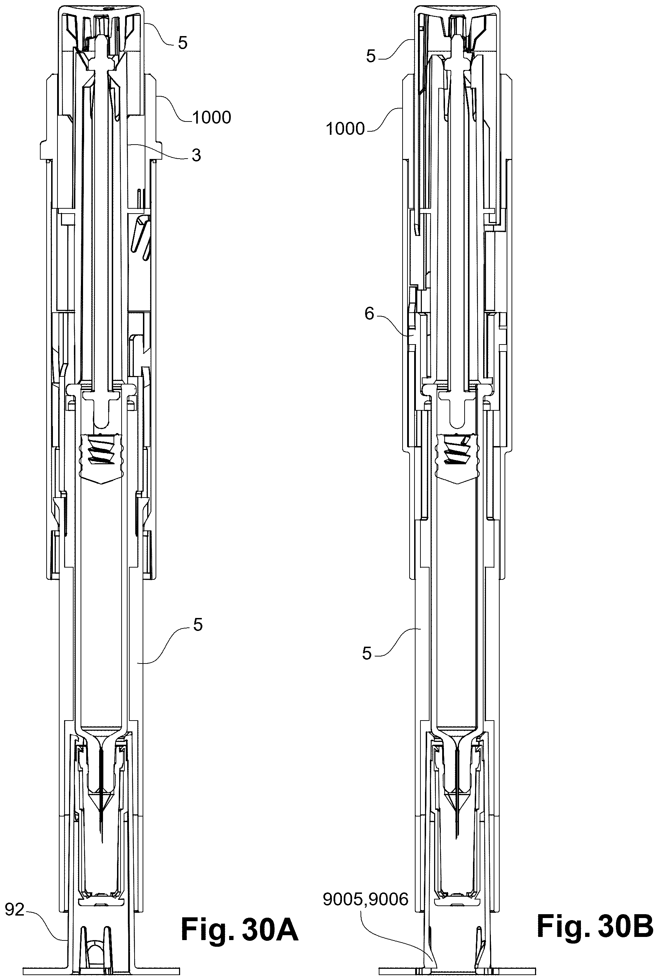

With reference to Figure 26, the remover 92 has a proximal portion extending within the needle cover 8 for engaging the needle shield 104 of the medical container 100 and a distal portion extending outside the needle cover 8 for allowing grasping of the remover 92 by the user. As illustrated in Figure 29, the remover 92 may have an outer flange 9001 at a distal end 9002, and may include one or more resilient legs 9003 distally extending between notches 9004 arranged through the inner sleeve 920 of the remover 92, at the distal end thereof. The resilient legs 9003 include an inner protrusion 9005, Figure 30B, whose proximal side may delimit an inclined surface 9006. This inner protrusion 9005 acts as a blocking member for retaining the detached needle shield 104 in the remover 92. The needle shield 104 may be evacuated through the opened distal end 9002 of the remover 92, by radially outwardly deforming the reslient legs 9003, so that the remover 92 can be re-used.

With reference to Figures 28A-28B, the distally protruding legs 521 of the actuation button 5 here need to be radially inwardly deflected so that the proximal abutment 522 of the actuation button 5 axially abuts against the distal end 1002 of the locker 1000. Besides, an axial blocking member 5500 of the actuation button 5 initially abuts against the proximal shoulder 1004 of the locker 1000 so that the actuation button 5 is initially axially blocked. Rotation of the locker 1000 from the locking position to the release position moves the proximal shoulder out of the path of the actuation button 5, which can thus be pressed by the user towards the actuation position.

The autoinjector 1 includes fasteners for axially securing the locker 1000 to the inner body 3. The fasteners may include the resilient leg 1006 whose proximal abutment 1009 may be configured for axially abutting against a distal side of the functional flange 311 of the inner body 3, Figure 31A, and the proximal end 20b of the outer body 2 axially abutting against the distal shoulder 1013 of the locker 1000 and/or the proximal stop 1008 of the locker 1000 axially abutting against the proximal side of the functional flange 311. As a result, the locker 1000 is axially fixed with respect to the outer body 2 and the inner body 3. The locker 1000 further has a radial blocking surface 1017, which may be an outer surface of the resilient leg 1006, configured for radially abutting against an inner surface of the outer body 2 when the locker 1000 is inserted within the outer body 2. This radial abutment prevents deformation of the resilient leg 1006, such that the locker 1000 alone cannot be dismantled. The actuation button 5 may also be provided with a radial blocking surface 5002, which may be arranged at an inner surface of the distal legs 521, for radially abutting against the inner body 3, Figure 31B. Therefore, this avoids disassembly of the actuation button 5 from the locker 1000.

With reference to Figure 32A, the locker 1000 includes an axial blocking member 1018 which may be the distal end of the distal legs 1003 for axially abutting against the needle cover 8 when the locker 1000 is in the locking position. Therefore, the needle cover 8 cannot inadvertently move to the retracted position, for instance because of an impact if dropped by the user. When the locker 1000 is rotated from the locking position to the release position, Figure 32B, the axial blocking member 1018 no longer extends on the axial path of the needle cover 8 so that the needle cover 8 can move towards the retracted position.

Indicators are provided for indicating the angular position of the locker 1000 to the user. The indicators may include the outer protrusion 1012 of the locker 1000, and a corresponding outer protrusion 2001 arranged at the proximal end 20b of the outer body 2. When the outer protrusions 1012, 2001 are axially offset, Figure 33A, the locker 1000 is in the locking position. When the outer protrusions 1012, 2001 are axially aligned, Figure 33B, the locker 1000 is in the release position.

The inner body 3 may include a tactile indicator 3001 which may be in the form of a radial protrusion arranged on an edge of the functional flange 311, Figures 34A-34B. The resilient leg 1006 of the locker 1000 has to pass over this radial protrusion 3001 when the locker 1000 is rotated from the locking position, Figure 34A, to the release position, Figure 34B, thereby providing tactile feedback to the user.

The assembly is similar to the previously described assembly, except that the power-pack here includes the locker 1000, and that no push-back step is required since there is no cap 91 and the remover 92 is directly positioned in engagement with the needle shield 104. The power-pack is assembled by snap-fitting the actuation button 5 in the locker 1000, snap-fitting the inner body 3 in the locker 1000, inserting the injection spring 37 in the inner body 3 and snap-fitting the plunger rod 4 in the inner body 3. The operation of the autoinjector 1 according to Figures 26-34B is similar to the operation of the autoinjector 1 of Figures 1-25C, except that the user here first has to rotate the locker 1000 from the locking position to the release position, and just has to pull the remover 92 instead of pulling the cap 91.

It is readily understandable from the above description that the autoinjector 1 of the invention is compact and easy to handle due to its small sized housing and its actuation button 5. Less raw material is required for manufacturing the autoinjector 1. This results in an improved sustainability. Besides, the autoinjector 1 reduces risks of skin pinching thanks to a long needle cover 8. It is to be understood that the present invention is not limited to the embodiments described above and illustrated in the drawings; rather, the skilled person will recognize that many changes and modifications may be made within the scope of the appended claims.

## Claims

1. Autoinjector (1) for automatic injection of a product into an injection site, said autoinjector (1) comprising :
an outer body (2) extending along a longitudinal axis A and configured for receiving a medical container (100) having a barrel (101) defining a reservoir for containing a medical product, said barrel (101) having a distal end (102) provided with an injection needle (103) sealed by a needle shield (104), and an opened proximal end provided with a radial flange (106),
a needle cover (8) axially movable along the longitudinal axis A between an initial position, a retracted position, proximally located with respect to the initial position, and a safety position, distally located with respect to the retracted position, in which the needle cover (8) shields the injection needle (103),
a holding ring (6) arranged within the outer body (2) for supporting the medical container (100), the holding ring (6) being axially movable between an initial position and a pricking position,
wherein the holding ring (6) is axially guided by the needle cover (8).

2. Autoinjector (1) according to the preceding claim, wherein the holding ring (6) includes one or more guiding members (650, 651) configured for complementarily engaging one or more guiding members (84) of the needle cover (8) such that these guiding members (650, 84) form a cylinder.

3. Autoinjector (1) according to any one of the preceding claims, wherein the needle cover (8) is axially guided by the outer body (2).

4. Autoinjector (1) according to any one of the preceding claims, wherein the autoinjector (1) includes a plunger rod (4) axially movable between an initial position and an injection end position, distally located relative to the initial position, the plunger rod (4) being configured to push a stopper (105) inside the barrel (101) in order to expel the medical product, an inner body (3) including a retainer for retaining the plunger rod (4) in the initial position, and an actuation button (5), axially movable with respect to the inner body (3) between an initial position and an actuation position in which the actuation button (5) engages the retainer of the inner body (3) to release the plunger rod (4) such that the plunger rod (4) can move to the injection end position.

5. Autoinjector (1) according to the preceding claim, wherein the inner body (3) is arranged within the outer body (2).

6. Autoinjector (1) according to any one of the preceding claims 4-5, wherein the actuation button (5) is arranged at a proximal end (20a) of the outer body (2).

7. Autoinjector (1) according to any one of the preceding claims 4-6, wherein the autoinjector (1) includes a provisional proximal blocking surface (312) configured for axially abutting against the actuation button (5) to provisionally block the actuation button (5) in the initial position.

8. Autoinjector (1) according to any one of the preceding claims 4-7, wherein the actuation button (5) includes push openings (54).

9. Autoinjector (1) according to any one of the preceding claims 4-8, wherein the inner body (3) includes a distal stop (35) for pushing the medical container (100) against the holding ring (6).

10. Autoinjector (1) according to any one of the preceding claims, wherein the autoinjector (1) includes a cam (7), rotatably mounted within the outer body (2) between a first angular position, a second angular position, and a third angular position, the cam (7) including a first track (73) engaged by the sequencing feature (86) of the needle cover (8) and a second track (74) engaged by the sequencing feature (64) of the holding ring (6).

11. Autoinjector (1) according to the preceding claim, wherein the cam (7) includes an axial blocking member (72) configured for preventing axial movement of the actuation button (5) to the actuation position as long as the cam (7) is in the first angular position.

12. Autoinjector (1) according to any one of the preceding claims, wherein the outer body (2) is made of a single piece.

13. Autoinjector (1) according to any one of the preceding claims, wherein the needle cover (8) mainly extends beyond the distal end (20a) of the outer body (2).

14. Autoinjector (1) according to any one of the preceding claims, wherein the autoinjector (1) includes a cap (91) which is removably attached to a distal end (20a) of the outer body (2), and the cap (91) extends over half the axial length of the needle cover (8).

15. Autoinjector (1) according to any one of the preceding claims, wherein the autoinjector (1) includes a cap (91) removably attached to a distal end (20a) of the outer body (2), and a remover (92) arranged within the cap (91) for removing the needle shield (104) when the cap (91) is withdrawn from the outer body (2), the cap (91) and the remover (92) including sequencing features (913, 925) configured for allowing removal of the cap (91) first and then removal of the needle shield (104) when the cap (91) is detached from the outer body (2).
